# FASCICULE DE BREVET EUROPEEN

(11) **EP 1 244 464 B1**
(45) Date de publication et mention de la délivrance du brevet: **03.03.2004**
(21) Numéro de dépôt: 00988909.8
(22) Date de dépôt: 14.12.2000
(51) Int. Cl.: A61K 35/78, A61K 7/48, A61P 17/16

(54) **EXTRAIT DE CELLULES DEDIFFERENTIEES D'UN VEGETAL DU GENRE LEONTOPODIUM ET COMPOSITIONS LE CONTENANT**
EXTRAKT VON UNDIFFERENZIERTEN ZELLEN EINER PFLANZE DER GATTUNG LEONTOPODIUM UND IHN ENTHALTENDE ZUSAMMENSETZUNGEN
EXTRACT OF DEDIFFERENTIATED CELLS FROM A PLANT BELONGING TO THE GENUS LEONTOPODIUM AND COMPOSITIONS CONTAINING SAME

(30) Priorité: 23.12.1999 FR 9916379
(43) Date de publication de la demande: 02.10.2002
(73) Titulaire: L'OREAL, 75008 Paris (FR)
(72) Inventeur: MARTIN, Richard, F-37210 Rochecorbon (FR); BELCOUR-CASTRO, Béatrice, F-37520 La Riche (FR); HILAIRE, Pascal, F-37210 Vouvray (FR); ROZOT, Roger, F-77400 Lagny-sur-Marne (FR)
(74) Mandataire: Allab, Myriam
(86) Numéro de dépôt international: PCT/FR2000/003529
(87) Numéro de publication internationale: WO 2001/047538

(56) Documents cités:
- FR-A- 2 731 162
- FR-A- 2 734 478
- "EDELWEISS EXTRACT FOR SUN PROTECTION" INSIDE COSMETICS, août 1998 (1998-08), page 35 XP000939189
- DATABASE BIOSIS [en ligne] BIOSCIENCES INFORMATION SERVICE, PHILADELPHIA, PA, US; 1989 HENNESSY D ET AL: "HYDROXYCINNAMIC ACID ESTERS FROM CELL SUSPENSION CULTURES AND PLANTS OF LEONTOPODIUM-ALPINUM" Database accession no. PREV198987096188 XP002147097 & PHYTOCHEMISTRY (OXFORD), vol. 28, no. 2, 1989, pages 489-490, ISSN: 0031-9422

## Description

La présente invention a pour objet dans son aspect le plus général un extrait de cellules dédifférenciées d'au moins un végétal du genre Leontopodium, un agent filtrant les rayonnements ultraviolets constitué d'au moins extrait de cellules dédifférenciées d'au moins un végétal du genre Leontopodium, une composition contenant au moins extrait de cellules dédifférenciées d'au moins un végétal du genre Leontopodium, l'utilisation dudit extrait comme agent filtrant les rayonnements ultraviolets et un procédé de traitement cosmétique faisant intervenir une composition comprenant ledit extrait.

Des extraits de végétal du genre Leontopodium sont proposés dans l'art antérieur comme agent protecteur des rayonnements ultraviolets. La société Alban Muller International (France ) commercialise d'ailleurs un tel extrait. Selon le fournisseur, il s'agit d'un extrait obtenu à l'aide d'un solvant composé d'eau et de propylène glycol à partir de feuilles et de fleurs de plants d'Edelweiss cultivés *in vivo* (zone de haute montagne).

La très connue Edelweiss appartient au genre végétal Leontopodium. Les végétaux du genre Leontopodium sont des végétaux rares qui poussent spontanément à des altitudes dépassant les 2000 mètres, particulièrement dans les massifs alpin et/ou népalais. Ces plantes de par leur rareté sont protégées et leur ramassage fait l'objet d'une réglementation extrêmement sévère. Ceci, ajouté au fait que les conditions naturelles de culture de ces végétaux sont particulières, fait qu'il s'avère difficile de les cultiver de façon industrielle à tout le moins dans des conditions qui à la fois ne mettent pas en péril les espèces utilisées et maintiennent un coût d'exploitation raisonnable.

La culture de cellules dédifférenciées de végétaux du genre Leontopodium se présente alors comme une solution aux problèmes ci-dessus exposés, d'autant plus que cela apporte entre autre avantage le fait que la matière végétale utilisable n'est plus saisonnière mais accessible toute l'année et par tout temps, répondant ainsi aux critères quantitatifs de l'industrie. De plus de telle culture sont parfaitement reproductibles puisque cultivées en conditions parfaitement contrôlées de température, de pH, de milieu de culture et répondent aux critères industriels de qualité.

Par cellules végétales dédifférenciées, on entend toute cellule végétale ne présentant aucun caractère d'une spécialisation particulière et capable de vivre par elle-même et non en dépendance avec d'autres cellules. Ces cellules végétales dédifférenciées sont éventuellement aptes, sous l'effet d'une induction, à toute différenciation conforme à leur génome.
Selon la méthode de culture choisie, et en particulier selon le milieu de culture choisi, il est possible d'obtenir à partir d'un même explant des cellules végétales dédifférenciées présentant des caractères différents. En effet, une des originalités des cellules végétales obtenues *in vitro* est la très grande variabilité qui s'établit spontanément dans les cultures. Cette variabilité est exprimée dans les domaines physiologiques et biochimiques. *De facto*, la culture *in vitro* entraîne la rupture des réseaux cellulaires existant dans les tissus *in vivo*. Progressivement, au fil des repiquages, de nouveaux réseaux d'interactions entre ces cellules cultivées *in vitro* et leur nouvel environnement s'établissent de façon aléatoire. Ces nouvelles interactions sont sources de nouveaux métabolites et/ou de modulations d'expression de métabolites présents dans la plante.

Mais, rien dans l'art antérieur n'indique que des extraits de cellules végétales dédifférenciées conserveront les propriétés des extraits de cellules végétales obtenues à partir de plantes cultivées *in vivo*. Particulièrement, à propos des végétaux du genre Leontopodium, rien n'indique ni ne suggère que les propriétés filtrantes développées naturellement par une plante naturelle exposée du fait de son biotope à de grandes quantités de rayonnements ultraviolets seront conservées par des cellules dédifférenciées qui du fait des conditions de leur culture ne sont pas exposées à de grandes quantités de rayonnements ultraviolets et qui par nature ont perdu les caractères de spécialisation particulière des cellules naturelles.

Par culture *in vivo* on entend toute culture de type classique c'est à dire en sol à l'air libre ou en serre, ou encore hors sol.
Par culture *in vitro*, on entend l'ensemble des techniques connues de l'homme du métier qui permet de manière artificielle l'obtention d'un végétal ou d'une partie d'un végétal. La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo*.

La demanderesse a maintenant montré qu'il est possible d'obtenir des extraits de cellules dédifférenciées d'au moins un végétal du genre Leontopodium et que de tels extraits présentent des propriétés de filtration des rayonnements ultraviolets, qui en font un produit de choix utilisable comme agent filtrant les rayonnements ultraviolets.

L'invention a donc pour objet premier, à titre de produit nouveau, un extrait de cellules dédifférenciées d'au moins un végétal du genre Leontopodium.

Le genre Leontopodium appartient à la famille des Compositae qui compte plus de 1300 genres et 21000 espèces.
Le genre Leontopodium comporte environ 10 espèces parmi lesquelles on peut citer *Leontopodium alpinum, Leontopodium stracheyi, Leontopodium nivale, Leontopodium palibinianum* (ou *Leontopodium sibiricum*), *Leontopodium souliei, Leontopodium linearifolium, Leontopodium gnaphalioides*.

Ainsi, l'extrait de cellules dédifférenciées d'au moins un végétal du genre Leontopodium de l'invention est un extrait préparé à partir de cellules dédifférenciées issues d'au moins un végétal appartenant à une espèce choisie parmi *Leontopodium alpinum, Leontopodium stracheyi, Leontopodium nivale, Leontopodium palibinianum* (ou *Leontopodium sibiricum*), *Leontopodium souliei, Leontopodium linearifolium, Leontopodium gnaphalioides*.

Préférentiellement selon l'invention, l'extrait est préparé à partir de cellules dédifférenciées issues de végétal appartenant à l'espèce *Leontopodium alpinum* ou à l'espèce *Leontopodium stracheyl*.

Outre que l'extrait de cellules dédifférenciées d'au moins un végétal du genre Leontopodium présentent des propriétés de filtration des rayonnements ultraviolets, il présente, entre autres avantages que ceux décrits ci-dessus pour les celles dédifférenciées, celui de présenter un spectre d'absorption de la lumière strictement limité au rayonnement UV, sans absorption du rayonnement visible. Il s'agit là d'un avantage non négligeable par rapport à un extrait obtenu avec une plante entière qui présente une absorption dans le domaine du visible, entraînant une coloration de l'extrait le rendant difficilement utilisable directement, particulièrement en cosmétique. L'extrait de cellules dédifférenciées d'au moins un végétal du genre Leontopodium peut être utilisé directement sans étape de décoloration qui s'avèrent en général longues et coûteuses pour un résultat incomplet, l'extrait de plantes entières n'étant généralement pas totalement décoloré.

Par la suite le terme "cellules dédifférenciées de Leontopodium" doit s'entendre comme "cellules dédifférenciées d'au moins un végétal du genre Leontopodium". De même le terme "extrait de Leontopodium" doit s'entendre comme "extrait de cellules dédifférenciées de Leontopodium" et donc comme "extrait de cellules dédifférenciées d'au moins un végétal du genre Leontopodium".

L'extrait de cellules dédifférenciées d'au moins un végétal du genre Leontopodium, peut être préparé par toute méthode d'extraction connue de l'homme du métier.
On peut en particulier citer les extraits alcooliques, notamment éthanoliques et ou méthanoliques, les extraits hydroalcooliques, ou encore les extraits aqueux tels que ceux dont la méthode de préparation est décrite dans le brevet français n° 95-02379.

Préférentiellement, selon l'invention on utilise un extrait éthanolique.

Quelque soit la méthode de préparation de l'extrait celui-ci peut alors être lyophilisé pour une éventuelle conservation.

Un exemple de préparation d'extrait utilisable selon l'invention est donné par ailleurs dans les exemples.

Le rayonnement solaire est composé, entre autres, de rayonnement ultraviolet de type A de longueurs d'onde comprises entre 320 nm et 400 nm (UV-A), de rayonnements ultraviolets de type B de longueurs d'onde comprises entre 280 et 320 nm (UV-B) et de rayonnements ultraviolets de type C de longueurs d'onde comprises entre 200 et 280 nm (UV-C).
Les UV-B sont fortement énergétiques et peu pénétrants, faiblement représentés dans la lumière solaire, dépendants des variations climatiques (temps nuageux, couvert, etc.) et leur présence varie en fonction des heures de la journée (notion de pic (zénith)).
Les UV-A sont moins énergétiques que les UV-B mais plus pénétrants, présents en grande quantité dans la lumière solaire (au minimum 100 fois plus d'UV-A que d'UV-B), peu dépendants des variations climatiques et présents quelle que soit l'heure de la journée.
Les UV-C sont fortement énergétiques et peu pénétrants. Ils sont théoriquement stoppés par la couche d'ozone. Mais potentiellement ils peuvent être responsables de dégâts des acides nucléiques.

Il est connu que le rayonnement solaire est responsable d'effets bénéfiques sur la peau, comme par exemple le brunissement, mais qu'il est aussi susceptible d'induire des dommages de celle-ci, notamment dans le cas d'une peau dite "sensible" ou d'une peau continuellement exposée.

Au titre des bienfaits le brunissement, communément appelé bronzage, est un élément essentiel du système de défense de la peau. En effet en réponse aux rayonnements ultraviolets, les mélanocytes de la couche supérieure de l'épiderme synthétisent de la mélanine qui une fois incorporée aux kératinocytes constitue un filtre situé à la surface de la peau, filtre qui absorbe le rayonnement ultraviolet. Ceci a pour objectif de diminuer la quantité de rayonnement ultraviolet qui traverse les couches de la peau afin de l'empêcher d'atteindre les couches profondes et d'y causer des dommages néfastes pour la peau.

Au titre des effets nocifs, on sait qu'une exposition excessive de la peau aux rayonnements ultraviolets, aux rayonnements solaires en particulier, peut entraîner un changement dans l'élasticité de la peau, dans sa teneur en certains composés, et ainsi favoriser l'accélération du processus naturel de vieillissement de la peau. Ce processus de vieillissement accéléré ou prématuré dû aux rayonnements ultraviolets est généralement appelé photo-vieillissement ou vieillissement actinique ou dermatohéliose.
Les UV-B faiblement pénétrants atteignent principalement l'épiderme. Le rôle des UV-B a été clairement démontré dans l'induction de cancers de la peau UV-induits. Ils ont en effet comme principal chromophore les acides nucléiques en particulier l'acide désoxyribonucléique, au sein duquel ils induisent des lésions et/ou des mutations (Eller M.S., 1995, in Photodamage. 26-56, Blackwell ed.).

Or les végétaux du genre Leontopodium, du fait de l'altitude à laquelle ils poussent, sont fortement exposés aux rayonnements ultraviolets. De fait, au risque de les voir disparaître, ils doivent posséder des systèmes de protection à l'égard des dommages induits par les rayonnements ultraviolets.

Là encore après de long travaux la demanderesse a montré qu'un extrait de cellules dédifférenciées d'au moins un végétal du genre Leontopodium tel que défini précédemment, possède des propriétés filtrantes à l'égard des rayonnements ultraviolets.

Par « filtrantes à l'égard des rayonnements ultraviolet » on entend selon l'invention, le pouvoir d'arrêter partiellement ou totalement le rayonnement ultraviolet de type A et/ou B et/ou C.

Ainsi, l'invention a pour deuxième objet un agent filtrant les rayonnements ultraviolets caractérisé en ce qu'il est constitué d'au moins un extrait de cellules dédifférenciées d'au moins un végétal du genre Leontopodium, tel que défini précédemment.

De plus la demanderesse a montré qu'un extrait obtenu avec des cellules dédifférenciées de végétal du genre *Leontopodium alpinum* présente un spectre d'absorption couvrant l'ensemble du spectre ultraviolet (UV-A, UV-B et UV-C).

Ainsi, l'invention a pour troisième objet un agent filtrant les rayonnements ultraviolets de type A, B et C, caractérisé en ce qu'il est constitué d'au moins un extrait de cellules dédifférenciées d'au moins un végétal de l'espèce *Leontopodium alpinum*.

La demanderesse a encore montré qu'un extrait obtenu avec des cellules dédifférenciées de végétal du genre *Leontopodium stracheyi* présente un spectre d'absorption ne couvrant spécifiquement que la partie du spectre correspondant aux rayonnement ultraviolet de type B et C.

Ainsi, l'invention a pour quatrième objet un agent filtrant les rayonnements ultraviolets de type B et C, caractérisé en ce qu'il est constitué d'au moins un extrait de cellules dédifférenciées d'au moins un végétal de l'espèce *Leontopodium stracheyi*.
La figure 1 présente un spectre d'absorption du rayonnement ultraviolet par un extrait de l'invention réalisé sur plante entière à partir d'un végétal du genre *Leontopodium alpinum*.
La figure 2 présente un spectre d'absorption du rayonnement ultraviolet par un extrait de l'invention réalisé sur cellules dédifférenciées à partir d'un végétal du genre *Leontopodium alpinum*.
La figure 3 présente un spectre d'absorption du rayonnement ultraviolet par un extrait de l'invention réalisé sur cellules dédifférenciées à partir d'un végétal du genre *Leontopodium stracheyi*.

L'invention a pour cinquième objet une composition comprenant dans un milieu physiologiquement acceptable, au moins un extrait de cellules dédifférenciées du genre Leontopodium tel que défini précédemment.

L'invention a pour sixième objet une composition comprenant dans un milieu physiologiquement acceptable, au moins un agent filtrant les rayonnements ultraviolets caractérisé en ce qu'il est constitué d'au moins un extrait de cellules dédifférenciées d'au moins un végétal du genre Leontopodium, tel que défini précédemment.

L'invention a pour septième objet une composition comprenant dans un milieu physiologiquement acceptable, au moins un agent filtrant les rayonnements ultraviolets de type A, B et C, caractérisé en ce qu'il est constitué d'au moins un extrait de cellules dédifférenciées d'au moins un végétal de l'espèce *Leontopodium alpinum*.

L'invention a pour septième objet une composition comprenant dans un milieu physiologiquement acceptable, au moins un agent filtrant les rayonnements ultraviolet de type B et C, caractérisé en ce qu'il est constitué d'au moins un extrait de cellules dédifférenciées d'au moins un végétal de l'espèce *Leontopodium stracheyi*.

Par milieu physiologiquement acceptable, on comprend un milieu compatible avec la peau, les muqueuses, les ongles, les cheveux.
Quelque soit sa forme, la composition de l'invention peut être une composition cosmétique ou dermatologique.
Préférentiellement selon l'invention la composition est une composition cosmétique.

Quelque soit la forme de la composition selon l'invention, la quantité d'extrait d'au moins un végétal du genre Leontopodium contenue dans la composition est bien entendu fonction de l'effet recherché et peut donc varier dans une large mesure.

Pour donner un ordre de grandeur, la quantité d'extrait d'au moins un végétal du genre Leontopodium contenue dans la composition est en une quantité représentant de 0.001% à 20% du poids total de la composition et préférentiellement en une quantité représentant de 1% à 10% du poids total de la composition.

Bien entendu la composition de l'invention peut, en plus de l'agent filtrant constitué d'au moins un extrait de cellules dédifférenciées d'au moins un végétal du genre Leontopodium tel que défini précédemment, comprendre au moins un autre composé reconnu comme agent filtrant des rayonnements ultraviolets.

Ainsi, l'invention a pour huitième objet une composition filtrante, caractérisée en ce qu'elle comprend, dans un milieu physiologiquement acceptable, un extrait de cellules dédifférenciées d'au moins un végétal du genre Leontopodium et au moins un autre composé reconnu comme agent filtrant des rayonnements ultraviolets.

L'extrait de cellules dédifférenciées d'au moins un végétal du genre Leontopodium est un extrait comme décrit précédemment dans le texte.

L'autre composé reconnu comme agent filtrant des rayonnements ultraviolets est de préférence choisi parmi les filtres organiques et/ou les filtres minéraux.

Comme filtres organiques on peut notamment citer les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665ou encore les filtres organiques décrits dans la demande de brevet EP-A 0 487 404.

Comme filtres minéraux on peut notamment citer des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Comme exemples de filtres solaires complémentaires actifs dans l'UV-A et/ou l'UV-B, on peut citer :
l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné
le p-aminobenzoate de glycérol,
le salicylate d'homomenthyle,
le salicylate de 2-éthylhexyle,
le salicylate de triéthanolamine,
le salicylate de 4-isopropylbenzyle,
le 4-ter-butyl-4'-méthoxy-dibenzoylméthane,
le 4-isopropyl-dibenzoylméthane,
l'anthranilate de menthyle,
le 2-éthylhexyl-2-cyano-3,3'-diphénylacrylate,
l'éthyl-2-cyano-3,3'-diphénylacrylate,
l'acide 2-phényl benzimidazole 5-sulfonique et ses sels,
le 3-(4'-triméthylammonium)-benzylidèn-boman-2-on-méthylsulfate,
le 2-hydroxy-4-méthoxybenzophénone,
le 2-hydroxy-4-méthoxybenzophénone-5-sulfonate,
le 2,4-dihydroxybenzophénone,
le 2,2',4,4'-tétrahydroxybenzophénone,
le 2,2'-dihydroxy-4,4'diméthoxybenzophénone,
le 2-hydroxy-4-n-octoxybenzophénone,
le 2-hydroxy-4-méthoxy-4'-méthylbenzophénone,
l'acide α-(2-oxoborn-3-ylidène)-tolyl-4-sulfonique et ses sels
le 3-(4'-sulfo)benzylidèn-bornan-2-one et ses sels,
le 3-(4'méthylbenzylidène)-d,l-camphre,
le 3-benzylidène-d,l-camphre,
l'acide benzène 1,4-di(3-méthylidène-10-camphosulfonique) et ses sels, l'acide urocanique,
la 2,4,6-tris-[p-(2'-éthylhexyl-1'-oxycarbonyl)anilino]-1,3,5-triazine, 2-[(p-(tertiobutylamido)anilino]-4,6-bis-[(p-(2'-éthylhexyl-1'-oxycarbonyl)anilino]-1,3,5-triazine,
la 2,4-bis {[4-2-éthyl-hexyloxy)]-2-hydroxy]-phenyl}-6-(4-méthoxy-phenyl)-1,3,5-triazine ;
le polymère de N-(2 et 4)-[(2-oxoborn-3-ylidèn)méthyl]benzyl]-acrylamide, l'acide 4,4-bis-benzimidazolyl-phénylèn-3,3',5,5'-tétrasulfonique et ses sels
le 2,2'-méthylèn-bis-[6-(2H-benzotriazol-2-yl)-4-(1,1,3,3-tétraméthylbutyl)phénol].
les polyorganosiloxanes à fonction malonate.

La quantité de composé reconnu comme agent filtrant des rayonnements ultraviolets, contenue dans la composition de l'invention est bien entendu fonction de l'effet recherché et peut donc varier dans une large mesure.

Pour donner un ordre de grandeur, la quantité d'agent filtrant des rayonnements ultraviolets autre que l'extrait d'au moins un végétal du genre Leontopodium contenue dans la composition est en une quantité représentant de 0,1% à 20% du poids total de la composition et préférentiellement en une quantité représentant de 0,5% à 10% du poids total de la composition.

Les compositions selon l'invention peuvent également contenir des agents de bronzage et/ou de brunissage artificiels de la peau (agents autobronzants), tels que par exemple de la dihydroxyacétone (DHA).

Les compositions de l'invention peuvent comprendre en outre des adjuvants cosmétiques classiques notamment choisis parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les homopolymères d'acide acrylique réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir ce ou ces éventuels composés complémentaires et/ou leurs quantités de manière telle que les propriétés avantageuses, en particulier le niveau de photoprotection, attachées intrinsèquement à l'association binaire conforme à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile, ou encore des compositions anhydres.

Cette composition peut se présenter en particulier sous forme d'émulsion, simple ou complexe (H/E, E/H, H/E/H ou E/H/E) telle qu'une crème, un lait, un gel ou un gel crème, de poudre, de composition solide, de pâtes souples et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de stick, de pâtes souples, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

L'invention a encore pour neuvième objet l'utilisation dans une composition ou pour la préparation d'une composition, dans un milieu physiologiquement acceptable, d'au moins un extrait de cellules dédifférenciées d'au moins un végétal du genre Leontopodium tel que défini précédemment, l'extrait ou la composition étant destinés à filtrer les rayonnements ultraviolets.

L'invention a aussi pour dixième objet l'utilisation dans une composition ou pour la préparation d'une composition destinée à filtrer les rayonnements ultraviolets, dans un milieu physiologiquement acceptable, d'un agent filtrant tel que défini précédemment.

Préférentiellement, ces compositions sont utilisées pour la protection de la peau et/ou des cheveux contre le rayonnement ultraviolet, en particulier le rayonnement solaire.

L'invention a en outre pour objet l'utilisation d'une quantité efficace d'une composition cosmétique telle que définie ci-dessus pour la préparation d'une composition Cosmétique destinée à protéger la peau ou les cheveux contre les effets des rayons UV consistant à appliquer sur ceux-ci.

Les exemples et compositions suivants illustrent l'invention sans la limiter aucunement. Dans les compositions les proportions indiquées sont des pourcentages en poids.

### Exemple 1 : Préparation de cellules dédifférenciées de Leontopodium stracheyi

Des feuilles de *Leontopodium stracheyi* sont prélevées et décontaminées avec des solutions saturées d'hypochlorite de sodium ou de calcium à température ambiante durant plusieurs minutes. Les tissus sont rincés à l'eau distillée stérile puis placés dans une solution d'éthanol dilué à 20%. Ils subissent 3 lavages à l'eau distillée stérile en fin de décontamination. Les feuilles sont alors découpées stérilement sous hotte à flux laminaire puis placés en boite de Pétri sur milieu nutritif gélosé de Murashige & Skoog dont la composition est pour 1 litre :

| | |
|---|---|
| Macro éléments de Skoog | 100,0 ml |
| Micro éléments de Skoog | 1,0 ml |
| Vitamines de Skoog | 2,0 ml |
| Fer EDTA | 10,0 ml |
| 2-4 D 10⁻⁴ M* | 10,0 ml |
| Kinétine 10⁻⁴ M | 0,6 ml |
| Saccharose | 30,0 g |
| Agar | 8,0 g |
| Eau distillée | qsp 1 litre |
| pH avant stérilisation | 5,8 UpH |

| | |
|---|---|
| * : Acide 2,4-dichlorophénoxyacétique 10⁻⁴ M (ou 2,4-D 10⁻⁴M) | |

Les macro éléments de Skoog ont pour composition, pour un litre :

| | |
|---|---|
| KNO₃ | 1900 mg |
| NH₄NO₃ | 1650 mg |
| MgSO₄, 7 H₂O | 370 mg |
| CaCl₂, 2 H₂O | 440 mg |
| KH₂PO₄ | 170 mg |

Les micro éléments de Skoog ont pour composition, pour un litre :

| | |
|---|---|
| CuSO₄, 5 H₂O | 0,025 mg |
| MnSO₄, 1 H₂O | 16,900 mg |
| KI | 0,830 mg |
| Na₂Mo0₄, 2 H₂O | 0,250 mg |
| ZnSO₄, 7 H₂O | 10,600 mg |
| H₃BO₃ | 6,200 mg |
| CoCl₂, 6 H₂O | 0,025 mg |

Les vitamines de Skoog ont pour composition, pour un litre :

| | |
|---|---|
| Myoinositol | 100,0 mg |
| Acide nicotinique | 0,5 mg |
| Pyridoxine | 0,5 mg |
| Thiamine | 0,1 mg |
| FeSO₄, 7 H₂O | 27,8 mg |
| Na₂ EDTA | 37,3 mg |

Les cals primaires apparaissent entre 2 et 5 semaines. Au fur et à mesure des repiquages, les cellules se stabilisent (aspect, couleur...) et sont transférées dans le même milieu de culture liquide (sans agar) en fermenteur ; Les cellules obtenues en milieu liquide sont récupérées par filtration sur toile de 50 à 100 µm suivant la taille des agrégats cellulaires.
Ces cellules sont placées à -20°C en congélation lente propice à la formation de cristaux intracellulaires de grande taille permettant de casser les cellules.

### Exemple 2 : Préparation de cellules dédifférenciées de Leontopodium alpinum

Des cellules dédifférenciées de *Leontopodium alpinum* ont été obtenues à partir de feuilles de *Leontopodium alpinum* selon le même protocole que celui utilisé à l'exemple 1.

### Exemple 3 : Préparation d'extraits éthanoliques :

Extrait A : 1,85 g poids frais de feuilles de *Leontopodium alpinum* congelés sont broyés (mortier, Potter...) dans 18,5 ml d'éthanol ;
Extrait B : 2,2 g poids frais de cals de *Leontopodium alpinum* congelés sont broyés (mortier, Potter...) dans 22 ml d'éthanol ;
Extrait C : 2,2 g poids frais de cals de *Leontopodium stracheyi* sont broyés (mortier, Potter...) dans 22 ml d'éthanol.

Chaque extrait est filtré sur filtre papier Prat Dumas N°5 pour éliminer les agrégats en suspension.
Chaque extrait est amené à sec sous vide (50 mBar/50°C) puis repris dans 4,4 ml d'éthanol (cals de *Leontopodium alpinum* et cals de *Leontopodium stracheyi*) ou 3,7 ml d'éthanol pour les feuilles de *Leontopodium alpinum*, soit environ 2 fois la masse de matériel végétal en volume d'éthanol. Cette opération permet d'éliminer entre autres les polysaccharides extraits dans la phase aqueuse amenée par les cellules fraîches.
L'extrait plante entière est d'un vert franc intense ce qui n'est pas le cas des extraits cellulaires issus de cals. Cette caractéristique de l'extrait de plante entière constitue un frein supplémentaire à son utilisation cosmétique en l'état. D'autre part, l'élimination des chlorophylles est une opération complexe au plan industriel.

### Exemple 4 : Mesure de la capacité d'absorption dans le visible et l'ultraviolet des extraits de l'exemple 3 :

Les extraits obtenus sont évalués pour leur capacité d'absorption dans le visible et l'ultraviolet sur un spectrophotomètre Perkin Elmer Lambda 2, en cuves quartz contre de l'éthanol.

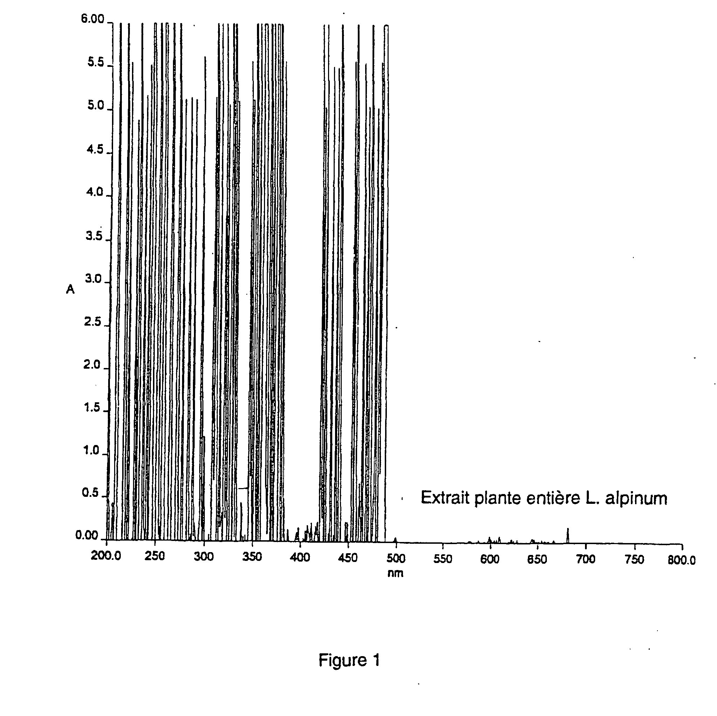

On constate que l'extrait de feuille de *Leontopodium alpinum* (extrait A, figure 1) présente spectre d'absorption des UV-A, -B et -C de 200 à 400 nm avec une bonne continuité (hormis de 370 à 400 nm).
On retrouve également une forte absorption entre 400 et 480 nm ainsi que des absorptions faibles au dessus de 570 nm. Cette absorption dans le visible est préjudiciable à l'utilisation industrielle d'un tel extrait.

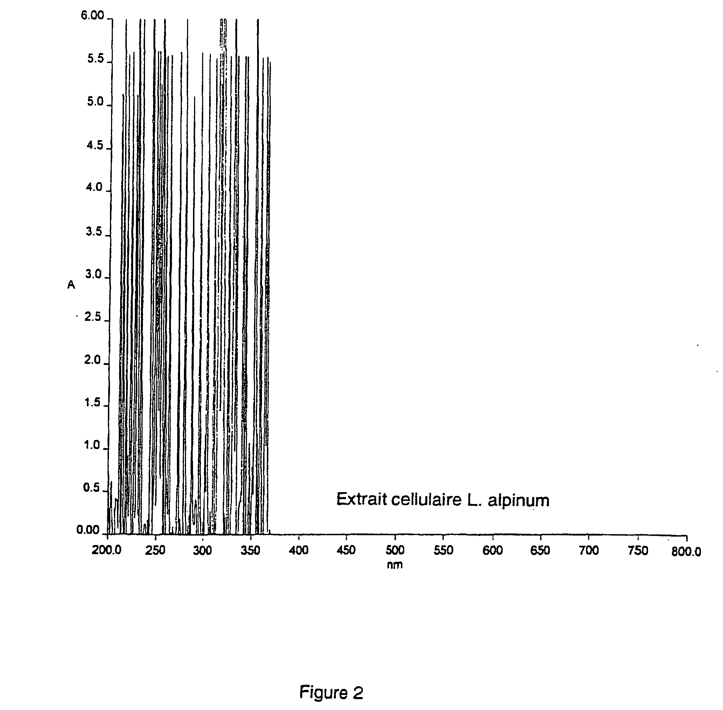

Le spectre d'absorption de l'extrait de cellules dédifférenciées de *Leontopodium alpinum* (extrait B, figure 2) couvre les UV-A, les UV-B et les UV-C jusqu'à environ 360 nm.

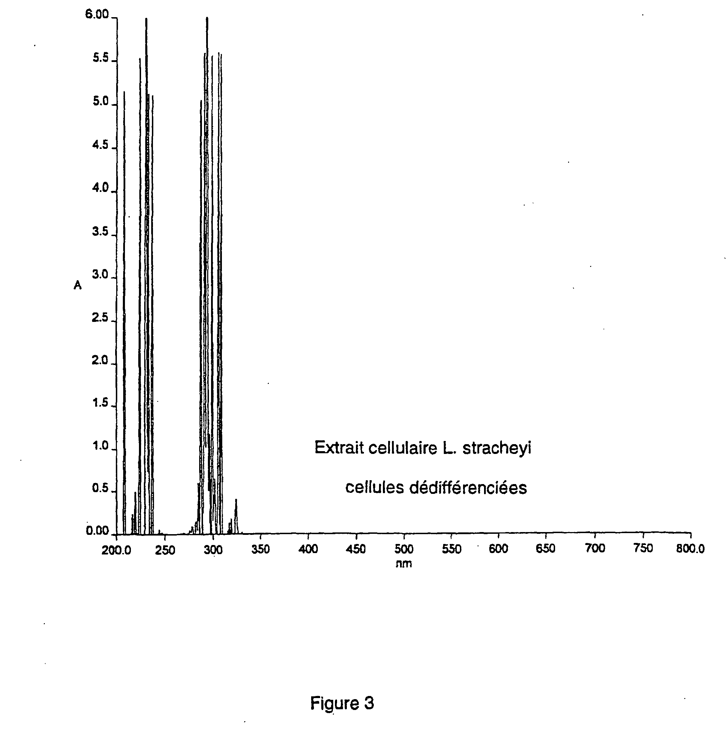

Le spectre d'absorption de l'extrait de cellules dédifférenciées de *Leontopodium stracheyi* (extrait C, figure 3) couvre uniquement les UV-B et -C.

On constate donc que les extraits éthanoliques de cellules dédifférenciées de végétaux du genre Leontopodium présentent des capacités d'absorption du rayonnement ultraviolet et ne présentent aucune absorption entre 400 et 480 nm et aucune absorption au dessus de 570 nm ce qui en fait de bons candidats pour une utilisation industrielle comme agent filtrant les rayonnements ultraviolets et particulièrement dans l'industrie cosmétique.

### Exemple 5 : Exemples de compositions illustrant l'invention. Ces compositions ont été obtenues par simple mélange des différents composants. Les quantités sont indiquées en pourcentage du total de la composition.

Composition 1 : Emulsion Huile dans Eau filtrant les UV-A, -B et ―C :
- Extrait de cellules dédifférenciées *Leontopodium alpinum* 5,0
- Mélange mono /distéarate de glycerol /-stéarate de polyéthylène glycol (100 OE) (ARLACEL 165 FL - ICI) 2,0
- Alcool stéarylique (LANETTE 18 - HENKEL) 1,0
- Acide stéarique d'huile de palme (STEARINE TP - STEARINERIE DUBOIS) 1,5
- Polydiméthylsiloxane (DOW CORNING 200 FLUID - DOW CORNING) 0,5
- Benzoate d'alcools en C12/C15 (WITCONOL TN -WITCO) 5,0
- Polydiméthyl/méthylsiloxane oxyéthyléné oxypropyléné en solution à 10 % dans D5 ( DC 5225 C -DOW CORNING ) 1,0
- Polyméthylphénylsiloxane ( MIRASIL PTM - RHODIA CHIMIE ) 3,0
- Triéthanolamine 0,5
- Glycérine 4,0
- Phosphate d'alcool hexadécylique, sel de potassium (AMPHISOL K - HOFFMAN LAROCHE) 1,0
- Acide polyacrylique (SYNTHALEN K - 3V) 0,3
- Hydroxypropylméthylcellulose (METHOCEL F4M -DOW CHEMICAL) 0,1
- Conservateurs qs
- Eau déminéralisée qsp 100,0 %
- Triéthanolamine qs pH =7

Composition 2 : Emulsion Eau dans Huile filtrant les UV-A,-B et ―C :
- Extrait de cellules dédifférenciées *Leontopodium alpinum* 1,5
- Poly diméthyl/méthylcétyl méthylsiloxane oxyéthyléné (ABIL EM 90D - GOLDSCHMIDT) 2,0
- Phényl triméthylsiloxy trisiloxane (DOW CORNING 556 COSMETIC grade fluid - DOW CORNING) 3,0
- Benzoate d'alcools en C12/C15 (WITCONOL TN - WITCO) 8,0
- Glycérine 5,0
- Sulfate de magnésium 0,7
- Conservateurs qs
- Eau déminéralisée qsp 100,0 %

Composition 3 : Emulsion Huile dans Eau filtrant les UV-B et ―C :
- Extrait de cellules dédifférenciées *Leontopodium stracheyi* 5,0
- Mélange mono /distéarate de glycerol / stéarate de polyéthylène glycol (100 OE) (ARLACEL 165 FL - ICI) 2,0
- Alcool stéarylique (LANETTE 18 - HENKEL) 1,0
- Acide stéarique d'huile de palme (STEARINE TP - STEARINERIE DUBOIS) 1,5
- Polydiméthylsiloxane (DOW CORNING 200 FLUID - DOW CORNING) 0,5
- Benzoate d'alcools en C12/C15 (WITCONOL TN -WITCO) 5,0
- Polydiméthyl/méthylsiloxane oxyéthyléné oxypropyléné en solution à 10 % dans D5 ( DC 5225 C -DOW CORNING ) 1,0
- Polyméthylphénylsiloxane ( MIRASIL PTM - RHODIA CHIMIE ) 3,0
- Triéthanolamine 0,5
- Glycérine 4,0
- Phosphate d'alcool hexadécylique, sel de potassium (AMPHISOL K - HOFFMAN LAROCHE) 1,0
- Acide polyacrylique (SYNTHALEN K - 3V) 0,3
- Hydroxypropylméthylcellulose (METHOCEL F4M -DOW CHEMICAL) 0,1
- Triéthanolamine qs pH =7
- Conservateurs qs
- Eau déminéralisée qsp 100,0 %

Composition 4 : Emulsion Eau dans Huile filtrant les UV-B et ―C :
- Extrait de cellules dédifférenciées *Leontopodium stracheyi* 1,5
- Poly diméthyl/méthylcétyl méthylsiloxane oxyéthyléné (ABIL EM 90D - GOLDSCHMIDT ) 2,0
- Phényl triméthylsiloxy trisiloxane (DOW CORNING 556 COSMETIC grade fluid - DOW CORNING) 3,0
- Benzoate d'alcools en C12/C15 (WITCONOL TN - WITCO) 8,0
- Glycérine 5,0
- Sulfate de magnésium 0,7
- Conservateurs qs
- Eau déminéralisée qsp 100,0 %

Composition 5 : Emulsion Huile dans Eau comprenant plusieurs filtres :
- Extrait de cellules dédifférenciée de *Leontopodium alpinum* 1,0
- Mélange mono /distéarate de glycerol / stéarate de polyéthylène glycol (100 OE) (ARLACEL 165 FL - ICI) 2,0
- Alcool stéarylique (LANETTE 18 - HENKEL) 1,0
- Acide stéarique d'huile de palme (STEARINE TP - STEARINERIE DUBOIS) 1,5
- Polydiméthylsiloxane (DOW CORNING 200 FLUID - DOW CORNING) 0,5
- Benzoate d'alcools en C12/C15 (WITCONOL TN -WITCO) 5,0
- Polydiméthyl/méthylsiloxane oxyéthyléné / oxypropyléné en solution à 10 % dans D5 (DC 5225 C -DOW CORNING) 1,0
- Polyméthylphénylsiloxane (MIRASIL PTM - RHODIA CHIMIE) 3,0
- Triéthanolamine 0,5
- 4-tert-butyl-4'-méthoxy dibenzoylméthane (PARSOL 1789-HOFFMANN LAROCHE) 1,0
- Octocrylène (UVINUL N539) 8,0
- Oxyde de titane (TITANIUM DIOXYDE MT-100 TV TAYCA) 2,5
- Glycérine 4,0
- Acide 1,4-bis-benzimidazoyl-phénylèn-3,3',5,5'-tétrasulfonique-sel de sodium 1,0
- Phosphate d'alcool hexadécylique, sel de potassium (AMPHISOL K - HOFFMAN LAROCHE) 1,0
- Acide polyacrylique (SYNTHALEN K - 3V) 0,3
- Hydroxypropylméthylcellulose (METHOCEL F4M -DOW CHEMICAL) 0,1
- Conservateurs qs
- Eau déminéralisée qsp 100,0 %
- Triéthanolamine qs pH =7

Composition 6 : Emulsion Eau dans Huile comprenant plusieurs filtres :
- Extrait de cellules dédifférenciée de *Leontopodium alpinum* 0,5
- Poly diméthyl/méthylcétyl méthylsiloxane oxyéthyléné (ABIL EM 90D - GOLDSCHMIDT ) 2,0
- Phényl triméthylsiloxy trisiloxane (DOW CORNING 556 COSMETIC grade fluid - DOW CORNING) 3,0
- Benzoate d'alcools en C12/C15 (WITCONOL TN - WITCO) 8,0
- Methylène bis-(tetraméthylbutyl hydroxyphényl benzotriazole) 5,0
- Drometrizole Trisiloxane 2,0
- 2,4-bis{[4-2-éthyl-hexyloxy)]-2-hydroxy]-phényl}-6-(4-méthoxy-phényl)-1,3,5-triazine 2,0
- Oxyde de titane (TITANIUM DIOXYDE MT100 TV - TAYCA) 3,0
- Glycérine 5,0
- Sulfate de magnésium 0,7
- Conservateurs qs
- Eau déminéralisée qsp 100,0 %

## Revendications

1. Extrait de cellules. dédifférenciées d'au moins un végétal du genre Leontopodium.

2. Extrait selon la revendication 1, **caractérisé en ce que** les cellules dédifférenciées sont des cellules provenant d'une espèce végétale choisie parmi les espèces *Leontopodium alpinum, Leontopodium stracheyi, Leontopodium nivale, Leontopodium palibinianum* (ou *Leontopodium sibiricum*), *Leontopodium souliei, Leontopodium linearifolium, Leontopodium gnaphalioides*.

3. Extrait selon l'une quelconque des revendications précédentes, **caractérisé en ce que** les cellules dédifférenciées sont des cellules provenant d'une espèce végétale choisie parmi les espèces *Leontopodium alpinum* ou *Leontopodium stracheyi*.

4. Extrait selon l'une quelconque des revendications précédentes, **caractérisé en ce qu'**il s'agit d'un extrait alcoolique ou d'un extrait hydroalcoolique ou encore d'un extrait aqueux.

5. Extrait selon la revendication précédente, **caractérisé en ce que** l'extrait alcoolique est un extrait éthanolique ou méthanolique.

6. Extrait selon l'une quelconque des revendications 4 ou 5, **caractérisé en ce que** l'extrait alcoolique est un extrait éthanolique.

7. Agent filtrant les rayonnements ultraviolets, **caractérisé en ce qu'**il est constitué d'au moins un extrait de cellules dédifférenciées d'au moins un végétal du genre Leontopodium tel que défini dans l'une quelconque des revendications précédentes.

8. Agent filtrant les rayonnements ultraviolets de type A, B et C, **caractérisé en ce qu'**il est constitué d'au moins un extrait de cellules dédifférenciées d'au moins un végétal de l'espèce *Leontopodium alpinum*.

9. Agent filtrant les rayonnements ultraviolet de type B et C, **caractérisé en ce qu'**il est constitué d'au moins un extrait de cellules dédifférenciées d'au moins un végétal de l'espèce *Leontopodium stracheyi*.

10. Composition, comprenant dans un milieu physiologiquement acceptable, au moins un extrait de cellules dédifférenciées d'au moins un végétal du genre Leontopodium tel que défini dans l'une quelconque des revendications 1 à 6.

11. Composition filtrant les rayonnements ultraviolets, comprenant dans un milieu physiologiquement acceptable, au moins un agent filtrant les rayonnements ultraviolets tel que défini dans l'une quelconque des revendications 7 à 9.

12. Composition, **caractérisée en ce qu'**elle comprend, dans un milieu physiologiquement acceptable, un extrait de cellules dédifférenciées d'au moins un végétal du genre Leontopodium et au moins un autre composé reconnu comme agent filtrant des rayonnements ultraviolets.

13. Composition selon la revendication 12, **caractérisée en ce que** l'agent filtrant les rayonnements ultraviolets est choisi parmi les filtres organiques et/ou les filtres minéraux.

14. Composition selon l'une quelconque des revendications 12 ou 13, **caractérisée en ce que** la quantité d'agent filtrant les rayonnements ultraviolets autre que l'extrait de cellules dédifférenciées d'au moins un végétal du genre Leontopodium est en une quantité représentant de 0,1% à 20% du poids total de la composition.

15. Composition selon la revendication 14, **caractérisée en ce que** la quantité d'agent filtrant les rayonnements ultraviolets autre que l'extrait de cellules dédifférenciées d'au moins un végétal du genre Leontopodium est en une quantité représentant de 0,5% à 10% du poids total de la composition.

16. Composition selon l'une quelconque des revendications 10 à 15, **caractérisée en ce que** la quantité d'extrait de cellules dédifférenciées d'au moins un végétal du genre Leontopodium contenue dans la composition est en une quantité représentant de 0.001% à 10% du poids total de la composition.

17. Composition selon la revendication 16, **caractérisée en ce que** la quantité d'extrait de cellules dédifférenciées d'au moins un végétal du genre Leontopodium contenue dans la composition est en une quantité représentant de 0,1% à 5% du poids total de la composition.

18. Composition selon l'une quelconque des revendications 10 à 17, **caractérisée en ce qu'**elle est destinée à un usage cosmétique.

19. Utilisation dans une composition ou pour la préparation d'une composition, d'au moins un extrait de cellules dédifférenciées d'au moins un végétal du genre Leontopodium tel que défini dans l'une quelconque des revendications 1 à 6, l'extrait ou la composition étant destinés à filtrer les rayonnements ultraviolets.

20. Utilisation dans une composition ou pour la préparation d'une composition, d'au moins un agent filtrant tel que défini dans l'une quelconque des revendications 7 à 9, l'agent filtrant ou la composition étant destinés à filtrer les rayonnements ultraviolets.

21. Utilisation selon l'une quelconque des revendications précédentes, pour la protection de la peau et/ou des cheveux contre les rayonnements ultraviolets.

22. Utilisation d'un extrait de cellules dédifférenciées d'au moins un végétal du genre Leontopodium pour la préparation d'une composition cosmétique selon la revendication 18, ladite composition étant destinée à protéger la peau et/ou les cheveux contre les effets des rayonnements ultraviolets.

## Claims

1. Extract of dedifferentiated cells of at least one plant of the genus Leontopodium.

2. Extract according to Claim 1, **characterized in that** the dedifferentiated cells are cells originating from a plant species chosen from the species *Leontopodium alpinum, Leon topodium stracheyi, Leontopodium nivale, Leontopodium palibinianum* (or *Leontopodium sibiricum), Leontopodium souliei, Leontopodium linearifolium* and *Leontopodium gnaphalioides*.

3. Extract according to any one of the preceding claims, **characterized in that** the dedifferentiated cells are cells originating from a plant species chosen from the species *Leontopodium alpinum* and *Leontopodium stracheyi*.

4. Extract according to any one of the preceding claims, **characterized in that** it is an alcoholic extract or an aqueous-alcoholic extract or an aqueous extract.

5. Extract according to the preceding claim, **characterized in that** the alcoholic extract is an ethanol or methanol extract.

6. Extract according to either one of Claims 4 and 5, **characterized in that** the alcoholic extract is an ethanol extract.

7. Agent for screening out ultraviolet radiation, **characterized in that** it consists of at least one extract of dedifferentiated cells of at least one plant of the genus Leontopodium as defined in any one of the preceding claims.

8. Agent for screening out ultraviolet radiation type A, B and C, **characterized in that** it consists of at least one extract of dedifferentiated cells of at least one plant of the species *Leontopodium alpinum*.

9. Agent for screening out ultraviolet radiation type B and C, **characterized in that** it consists of at least one extract of dedifferentiated cells of at least one plant of the species *Leontopodium stracheyi*.

10. Composition comprising, in a physiologically acceptable medium, at least one extract of dedifferentiated cells of at least one plant of the genus Leontopodium-as defined in any one of Claims 1 to 6.

11. Composition for screening out ultraviolet radiation, comprising, in a physiologically acceptable medium, at least one agent for screening out ultraviolet radiation as defined in any one of Claims 7 to 9.

12. Composition, **characterized in that** it comprises, in a physiologically acceptable medium, an extract of dedifferentiated cells of at least one plant of the genus Leontopodium and at least one other compound known as an agent for screening out ultraviolet radiation.

13. Composition according to Claim 12, **characterized in that** the agent for screening out ultraviolet radiation is chosen from organic screening agents and/or inorganic screening agents.

14. Composition according to either one of Claims 12 and 13, **characterized in that** the amount of agent for screening out ultraviolet radiation, other than the extract of dedifferentiated cells of at least one plant of the genus Leontopodium, is an amount representing from 0.1% to 20% of the total weight of the composition.

15. Composition according to Claim 14, **characterized in that** the amount of agent for screening out ultraviolet radiation, other than the extract of dedifferentiated cells of at least one plant of the genus Leontopodium, is an amount representing from 0.5% to 10% of the total weight of the composition.

16. Composition according to any one of Claims 10 to 15, **characterized in that** the amount of extract of dedifferentiated cells of at least one plant of the genus Leontopodium contained in the composition is an amount representing from 0.001% to 10% of the total weight of the composition.

17. Composition according to Claim 16, **characterized in that** the amount of extract of dedifferentiated cells of at least one plant of the genus Leontopodium contained in the composition is an amount representing from 0.1% to 5% of the total weight of the composition.

18. Composition according to any one of Claims 10 to 17, **characterized in that** it is intended for cosmetic use.

19. Use, in a composition or for preparing a composition, of at least one extract of dedifferentiated cells of at least one plant of the genus Leontopodium as defined in any one of Claims 1 to 6, the extract or the composition being intended to screen out ultraviolet radiation.

20. Use, in a composition or for preparing a composition, of at least one screening agent as defined in any one of Claims 7 to 9, the screening agent or the composition being intended to screen out ultraviolet radiation.

21. Use according to any one of the preceding claims, for protecting the skin and/or the hair against ultraviolet radiation.

22. Use of an extract of dedifferentiated cells of at least one plant of the genus Leontopodium, for preparing a cosmetic composition according to Claim 18, said composition being intended to protect the skin and/or the hair against the effects of ultraviolet radiation.

## Patentansprüche

1. Extrakt von undifferenzierten Zellen mindestens einer Pflanze der Gattung Leontopodium.

2. Extrakt nach Anspruch 1, **dadurch gekennzeichnet, dass** es sich bei den undifferenzierten Zellen um Zellen-handelt, die von einer Pflanzenart stammen, die unter den Spezies *Leontopodium alpinum, Leontopodium stracheyi, Leontopodium nivale, Leontopodium palibinianum* (oder *Leontopodium sibiricum*), *Leontopodium souliei, Leontopodium linearifolium* und *Leontopodium gnaphalioides* ausgewählt ist.

3. Extrakt nach einem der vorhergehenden Ansprüche, **dadurch gekennzeichnet, dass** die undifferenzierten Zellen Zellen sind, die von einer Pflanzenart stammen, die unter den Spezies *Leontopodium alpinum* oder *Leontopodium stracheyi* ausgewählt ist.

4. Extrakt nach einem der vorhergehenden Ansprüche, **dadurch gekennzeichnet, dass** es sich um einen alkoholischen Extrakt oder einen wässrig-alkoholischen Extrakt oder einen wässrigen Extrakt handelt.

5. Extrakt nach dem vorhergehenden Anspruch, **dadurch gekennzeichnet, dass** der alkoholische Extrakt ein Ethanolextrakt oder Methanolextrakt ist.

6. Extrakt nach einem der Ansprüche 4 oder 5, **dadurch gekennzeichnet, dass** der alkoholische Extrakt ein ethanolischer Extrakt ist.

7. UV-Filter, **dadurch gekennzeichnet, dass** es aus mindestens einem Extrakt von undifferenzierten Zellen mindestens einer Pflanze der Gattung Leontopodium nach einem der vorhergehenden Ansprüche besteht.

8. UV-Filter vom Typ A, B und C, **dadurch gekennzeichnet, dass** es aus mindestens einem Extrakt von undifferenzierten Zellen mindestens einer Pflanze der Spezies *Leontopodium alpinum* besteht.

9. UV-Filter vom Typ B und C, **dadurch gekennzeichnet, dass** es aus mindestens einem Extrakt von undifferenzierten Zellen mindestens einer Pflanze der Spezies *Leontopodium stracheyi* besteht.

10. Zusammensetzung, die in einem physiologisch akzeptablen Medium mindestens einen Extrakt von undifferenzierten Zellen mindestens einer Pflanze der Gattung Leontopodium nach einem der Ansprüche 1 bis 6 enthält.

11. Zusammensetzung, die UV-Strahlung filtert und die in einem physiologisch akzeptablen Medium mindestens ein UV-Filter nach einem der Ansprüche 7 bis 9 enthält.

12. Zusammensetzung, **dadurch gekennzeichnet, dass** sie in einem physiologisch akzeptablen Medium einen Extrakt von undifferenzierten Zellen mindestens einer Pflanze der Gattung Leontopodium und mindestens eine weitere Verbindung enthält, die als UV-Filter bekannt ist.

13. Zusammensetzung nach Anspruch 12, **dadurch gekennzeichnet, dass** das UV-Filter unter den organischen Filtern und/oder den anorganischen Filtern ausgewählt ist.

14. Zusammensetzung nach einem der Ansprüche 12 oder 13, **dadurch gekennzeichnet, dass** der Mengenanteil des UV-Filters, das von dem Extrakt von undifferenzierten Zellen mindestens einer Pflanze der Gattung Leontopodium verschieden ist, in einer Menge von 0,1 bis 20 % des Gesamtgewichts der Zusammensetzung enthalten ist.

15. Zusammensetzung nach Anspruch 14, **dadurch gekennzeichnet, dass** die Menge des UV-Filters, das von dem Extrakt von undifferenzierten Zellen mindestens einer Pflanze der Gattung Leontopodium verschieden ist, im Bereich von 0,5 bis 10 % des Gesamtgewichts der Zusammensetzung vorliegt.

16. Zusammensetzung nach einem der Ansprüche 10 bis 15, **dadurch gekennzeichnet, dass** der Extrakt von undifferenzierten Zellen mindestens einer Pflanze der Gattung Leontopodium, der in der Zusammensetzung enthalten ist, in einem Mengenanteil von 0,001 bis 10 % des Gesamtgewichts der Zusammensetzung vorliegt.

17. Zusammensetzung nach Anspruch 16, **dadurch gekennzeichnet, dass** der in der Zusammensetzung enthaltene Extrakt von undifferenzierten Zellen mindestens einer Pflanze der Gattung Leontopodium in einer Menge von 0,1 bis 5 % des Gesamtgewichts der Zusammensetzung vorliegt.

18. Zusammensetzung nach einem der Ansprüche 10 bis 17, **dadurch gekennzeichnet, dass** sie für eine kosmetische Verwendung vorgesehen ist.

19. Verwendung mindestens eines Extraktes von undifferenzierten Zellen mindestens einer Pflanze der Gattung Leontopodium nach einem der Ansprüche 1 bis 6 in einer Zusammensetzung oder zur Herstellung einer Zusammensetzung, wobei der Extrakt oder die Zusammensetzung dazu vorgesehen sind, UV-Strahlung zu filtern.

20. Verwendung mindestens eines Filters nach einem der Ansprüche 7 bis 9 in einer Zusammensetzung oder zur Herstellung einer Zusammensetzung, wobei das Filter oder die Zusammensetzung dazu vorgesehen sind, die UV-Strahlung zu filtern.

21. Verwendung nach einem der vorhergehenden Ansprüche zum Schutz der Haut und/oder der Haare gegen UV-Strahlung.

22. Verwendung eines Extrakts von undifferenzierten Zellen mindestens einer Pflanze der Gattung Leontopodium zur Herstellung einer kosmetischen Zusammensetzung nach Anspruch 18, wobei die Zusammensetzung dazu vorgesehen ist, die Haut und/oder die Haare gegen die Wirkungen der UV-Strahlung zu schützen.
